(19) **Europäisches Patentamt / European Patent Office / Office européen des brevets**

(11) **EP 3 133 056 B1**

(12) **EUROPEAN PATENT SPECIFICATION**

(45) Date of publication and mention
of the grant of the patent:
**02.01.2019 Bulletin 2019/01**

(51) Int Cl.:
**C07C 51/60** (2006.01)   **C07C 51/64** (2006.01)
**C07C 53/42** (2006.01)

(21) Application number: **15779479.3**

(22) Date of filing: **14.04.2015**

(86) International application number:
**PCT/JP2015/061504**

(87) International publication number:
**WO 2015/159893 (22.10.2015 Gazette 2015/42)**

(54) **PRODUCTION METHOD OF FATTY ACID CHLORIDE AND FATTY ACID CHLORIDE**

VERFAHREN ZUR HERSTELLUNG VON FETTSÄURECHLORID UND FETTSÄURECHLORID

PROCÉDÉ DE FABRICATION DE CHLORURE D'ACIDE GRAS ET CHLORURE D'ACIDE GRAS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.04.2014 JP 2014082493**

(43) Date of publication of application:
**22.02.2017 Bulletin 2017/08**

(73) Proprietor: **NOF Corporation
Shibuya-ku
Tokyo 150-6019 (JP)**

(72) Inventors:
• **FUJITA Hiroya
Amagasaki-shi
Hyogo 660-0095 (JP)**
• **OHARA Shinji
Amagasaki-shi
Hyogo 660-0095 (JP)**

(74) Representative: **Hoffmann Eitle
Patent- und Rechtsanwälte PartmbB
Arabellastraße 30
81925 München (DE)**

(56) References cited:
**JP-A- H01 211 547      JP-A- S63 316 753
JP-A- 2014 058 458**

**Description**

TECHNICAL FIELD

The present invention relates to a production method of a fatty acid chloride by producing using phosphorus trichloride and a fatty acid, and a fatty acid chloride obtained.

BACKGROUND ART

A fatty acid chloride is used in the synthesis of an alkyl ketene dimer, an organic peroxide, a surfactant, an intermediate of medicine or the like. In general, the fatty acid chloride can be obtained by reacting a fatty acid with a chlorinating agent. As the chlorinating agent, for example, phosphorus trichloride or carbonyl chloride is used.

The production method of a fatty acid chloride using phosphorus trichloride has an advantage in that the production is relatively simple, but on the other hand it has a disadvantage in that a phosphorus compound, for instance, an inorganic phosphorus compound, for example, phosphorous acid by-produced in the reaction or unreacted phosphorus trichloride or an organic phosphorus compound by-produced by a reaction with an alcohol as a low material impurity, or an unreacted fatty acid is present in the fatty acid chloride as an impurity.

In order to solve the problem of impurity, a method of adding an additive capable of forming a complex with the phosphorus compound or a method of removing the phosphorus compound and fatty acid by distillation has been taken.

In Patent Document 1 (JP-A-6-41000), a production method for obtaining an aliphatic carboxylic acid chloride containing no phosphorus component by adding a metal halide compound to form a metal complex with the phosphorus compound by-produced containing phosphorus acid as the main component and then conducting distillation or the like. This technique makes it possible to obtain a fatty acid chloride containing no phosphorus component in high purity and in high yield, but as to the hue, coloration is recognized and further there is a problem in that the fatty acid chloride purified is colored with the lapse of time.

Thus, in Patent Document 2 (JP-A-11-255703), a production method of an acid chloride in which the phosphorus compound is reduced and which has stable hue by adding magnesium oxide, N,N-dimethylformamide or the like as the additive capable of forming a complex with the phosphorus compound and conducting distillation. However, in the technique, methacrylic acid having a polymerizable group is used and since its behaver is different from that of fatty acid, the problem of coloration of the fatty acid chloride purified with the lapse of time is not solved.

Further, in Patent Document 3 (WO2005/032509), there is described that an N-acylaspartic acid which is not colored in yellow even during preservation at high temperature is obtained by using a fatty acid chloride containing a total phosphorus content and an organic phosphorus content in the specified amounts. In this technique, the total phosphorus content and the organic phosphorus content are specified in order to prevent the coloration, but others are not specified. Further, although hue change with the lapse of time of the N-acylaspartic acid is determined under conditions of 50°C for 30 days, hue change with the lapse of time of the fatty acid chloride per se is not evaluated.

Patent Document 4 describes a process for producing carboxylic acid halides having the formula $RC(=O)X$ wherein R represents an organic residue with more than seven carbon atoms and X is chlorine or bromine by reacting carboxylic acid with phosphorus chloride or phosphorus bromide. Then the carboxylic acid amide hydrochloride or hydrobromide phase is separated. Patent Document 5 describes a method for purifying an acyl halide wherein volatile components have been removed in advance using activated carbon.

Patent Document 6 describes the preparation of high purity aliphatic dicarboxylic acid dichloride from an aliphatic dicarboxylic acid having a melting point of 76°C or more. Then phosphorus trichloride is charged and then an aliphatic dicarboxylic acid is charged.

PRIOR ART DOCUMENT

PATENT DOCUMENT

Patent Document 1: JP-A-6-041000
Patent Document 2: JP-A-11-255703
Patent Document 3: WO2005/032509
Patent Document 4: JP-A-S63-316753
Patent Document 5: JP-A-H01-211547
Patent Document 6: JP-A-2014-058458

## SUMMARY OF THE INVENTION

### PROBLEMS THAT THE INVENTION IS TO SOLVE

A problem of the invention is to provide a production method of a fatty acid chloride which is free from turbidity and has stable hue with the lapse of time by a simple production method.

### MEANS FOR SOLVING THE PROBLEMS

As a result of the intensive investigations, the inventors have found that a fatty acid chloride which is produced according to the steps described below is free from turbidity and has stable hue with the lapse of time to complete the invention.

Specifically, the invention includes the following items.

(1) A production method of a fatty acid chloride comprising performing the following Step 1, Step 2 and Step 3 in this order:

Step 1:
a step of reacting a fatty acid having from 8 to 22 carbon atoms with from 1/3 to 2/3 molar equivalent of phosphorus trichloride relative to the fatty acid to prepare a fatty acid chloride, and separating, by standing, a layer containing phosphorous acid by-produced to obtain a reaction product;
Step 2:
a step of purifying the reaction product obtained in the Step 1 so as to set a phosphorus content of the phosphorous acid by-produced to 0.20% by weight or less;
Step 3:

a step of adding from 0.03 to 0.07% by weight of phosphorus trichloride in terms of a phosphorus content to the purified product obtained in the Step 2,
wherein, in the Step 2, the purification is performed by treating the reaction product obtained in the Step 1 at a temperature from 80 to 230°C and under a pressure from $133.3 \times 10^{-1}$ to $133.3 \times 10^{1}$ Pa using a thin film distiller to distil away the phosphorus trichloride in the reaction product outside a system and to evaporate the fatty acid chloride and condense the fatty acid chloride by an internal condenser, thereby separating the phosphorous acid by-produced which is a non-evaporative component, or
wherein, in the Step 2, the purification is performed by treating the reaction product obtained in the Step 1 at a temperature from 40 to 90°C and under a pressure from $133.3 \times 10^{-1}$ to $133.3 \times 10^{1}$ Pa using a thin film distiller to distil away the phosphorus trichloride in the reaction product outside a system without evaporating the fatty acid chloride, thereby removing the phosphorous acid by-produced dissolved in the phosphorus trichloride together with the distillation away of the phosphorus trichloride from the fatty acid chloride.

(2) The method as described in (1), wherein a phosphorus content of an inorganic phosphorus compound contained in the fatty acid chloride obtained in the Step 3 is from 0.03 to 0.27% by weight and a phosphorus content of an organic phosphorus compound contained in the fatty acid chloride obtained in the Step 3 is from 0.04 to 0.10% by weight.

### ADVANTAGE OF THE INVENTION

According to the invention, a fatty acid chloride which is free from turbidity and has stable hue with the lapse of time can be obtained by a simple production method.

### MODE FOR CARRYING OUT THE INVENTION

The invention will be described in detail hereinafter.

The invention relates to a production method of a fatty acid chloride comprising performing Steps 1 to 3 in this order. Each of the steps is described below.

The fatty acid used in the invention is a saturated or unsaturated fatty acid having from 8 to 22 carbon atoms. As specific examples, a fatty acid, for example, lauric acid, myristic acid, palmitic acid, isopalmitic acid, stearic acid, isostearic acid, oleic acid, arachidic acid or behenic acid, and a mixed fatty acid, for example, palm oil fatty acid, palm nucleus fatty acid or beef tallow fatty acid can be used. Lauric acid, myristic acid, palm oil fatty acid and palm nucleus fatty acid are preferred, and lauric acid and palm oil fatty acid are particularly preferred.

<Step 1>

Step 1 is a step of reacting a fatty acid having from 8 to 22 carbon atoms with from 1/3 to 2/3 molar equivalent of phosphorus trichloride relative to the fatty acid to prepare a fatty acid chloride, and separating, by standing, a layer containing phosphorous acid by-produced to obtain a reaction product.

The amount of phosphorus trichloride is from 1/3 to 2/3 molar equivalent, preferably from 1.1/3 to 1.8/3 molar equivalent, more preferably from 1.3/3 to 1.7/3 molar equivalent, relative to the fatty acid.

The reaction temperature between phosphorus trichloride and a fatty acid is preferably from 40 to 90°C. The reaction temperature is more preferably 45°C or more, and still more preferably 50°C or more. Also, the reaction temperature is more preferably 80°C or less, and still more preferably 70°C or less.

The addition of phosphorus trichloride is preferably conducted stepwise and more preferably conducted dropwise. After the pouring of phosphorus trichloride, ripening is performed preferably for 0.5 to 5 hours, more preferably for 1 to 4 hours, and still more preferably for 1 to 3 hours.

After the ripening, the stirring is terminated, followed by allowing to stand. Thus, a fatty acid chloride layer of an upper layer and a by-produced phosphorous acid layer of a lower layer are separated. The by-produced phosphorous acid of the lower layer is removed to obtain the fatty acid chloride layer of the upper layer. The time for the standing is preferably from 2 to 12 hours, more preferably from 3 to 11 hours, and still more preferably from 4 to 10 hours. Also, the temperature for the standing is preferably from 40 to 90°C, more preferably from 45 to 80°C, and still more preferably from 50 to 70°C.

<Step 2>

Step 2 is a step of purifying the reaction product obtained in Step 1 so as to set a phosphorus content of the phosphorous acid by-produced to 0.20% by weight or less.

In the reaction product obtained in Step 1, since the phosphorous acid by-produced is dissolved in the remaining phosphorus trichloride, it is necessary to distil away the phosphorus trichloride in order to remove the phosphorous acid by-produced.

As the purifying method, a method using thin film distillation or thin film topping is used. The topping as used herein denotes a step of distilling away the phosphorus trichloride in the fatty acid chloride.

The thin film distillation includes forming a thin film using a thin film distiller, and heating the thin film to distil away the phosphorus trichloride in the reaction product outside a system and to evaporate the fatty acid chloride and condense the fatty acid chloride by an internal condenser. Since the phosphorous acid by-produced is a non-evaporative component, it is separated from the fatty acid chloride. By treating according to the step, the phosphorous acid by-produced can be removed together with the distillation away of the phosphorus trichloride. The method for forming the thin film is not particularly limited, and any conventionally known method, for example, a falling type, a centrifugal type, a stirring type, a rotary type, a blade type or a rising type can be adopted.

The conditions of the thin film distillation varies depending on a fatty acid as the raw material, and the temperature is from 80 t0 230°C. When the temperature is less than 80°C, the yield becomes low and it is not effective. When it exceeds 230°C, there is a danger that decomposition of the fatty acid chloride due to heat or deterioration of hue may occur.

Also, in order to enhance efficiency of the distillation of the fatty acid chloride, the distillation is performed under a reduced pressure. By controlling the degree of vacuum, the phosphorus trichloride can be effectively distilled away even at a low temperature, and the decomposition of fatty acid chloride due to heat can be prevented. The pressure of the step is from $133.3 \times 10^{-1}$ to $133.3 \times 10^1$ Pa, preferably from 66.7 to 666.5 Pa, and more preferably from 133.3 to 399.9 Pa. When the pressure exceeds $133.3 \times 10^1$ Pa, the distillation away of the phosphorus trichloride may become insufficient so that the removal of the phosphorous acid by-produced may become difficult.

Further, in order to enhance efficiency of the distillation of the fatty acid chloride, nitrogen gas may be injected in the thin film distiller.

The thin film topping includes forming a thin film using a thin film distiller, and heating the thin film to distil away the phosphorus trichloride in the reaction product outside a system and since the phosphorous acid by-produced is dissolved in the phosphorus trichloride, when the phosphorus trichloride is distilled away, the phosphorous acid by-produced is also removed from the fatty acid chloride. By treating according to the step, the phosphorous acid by-produced can be removed together with the distillation away of the phosphorus trichloride. The method for forming the thin film is not particularly limited, and any conventionally known method, for example, a falling type, a centrifugal type, a stirring type, a rotary type, a blade type or a rising type can be adopted.

As to the conditions of the thin film topping, the temperature is from 40 to 90°C. When the temperature is less than 40°C, the distillation away of the phosphorus trichloride is insufficient so that the removal of the phosphorous acid by-produced may become difficult. When the temperature exceeds 90°C, hue of the fatty acid chloride may degrade and the yield may decrease due to distillation away of the fatty acid chloride (non-evaporative component). The temperature at the thin film topping is preferably 45°C or more, and more preferably 50°C or more. Also, the temperature at the thin film topping is preferably 80°C or less, and more preferably 70°C or less.

Also, in order to enhance efficiency of the distillation away of the unreacted phosphorus trichloride, the topping is performed under a reduced pressure. By controlling the degree of vacuum, the phosphorus trichloride can be effectively distilled away even at a low temperature, and the decomposition of fatty acid chloride due to heat can be prevented. The pressure of the step is from $133.3 \times 10^{-1}$ to $133.3 \times 10^{1}$ Pa, preferably from 100.0 to 999.8 Pa, and more preferably from 133.3 to 666.5 Pa. When the pressure exceeds $133.3 \times 10^{1}$ Pa, the distillation away of the phosphorus trichloride may become insufficient so that the removal of the phosphorous acid by-produced may become difficult. When the pressure is less than $133.3 \times 10^{-1}$ Pa, the fatty acid chloride distilled away likely to increase.

Further, in order to enhance efficiency of the distillation away of the unreacted phosphorus trichloride, nitrogen gas may be injected in the thin film distiller.

<Step 3>

Step 3 is a step of adding from 0.03 to 0.07% by weight of phosphorus trichloride in terms of a phosphorus content to the purified product obtained in Step 2.

Thus, it is possible that a phosphorus content of an inorganic phosphorus compound contained in the product obtained is set from 0.03 to 0.27% by weight and a phosphorus content of an organic phosphorus compound contained in the product obtained is set from 0.04 to 0.2% by weight.

In the fatty acid chloride before performing Step 3, by setting the phosphorus content of the phosphorous acid by-produced to 0.20% by weight or less, turbidity of the fatty acid chloride and deterioration of hue with the lapse of time can be inhibited.

The phosphorus content of the inorganic phosphorus compound contained in the fatty acid chloride obtained by performing Step 3 is from 0.03 to 0.27% by weight. The phosphorus content exceeding 0.27% by weight may become a factor for causing white turbidity of the fatty acid chloride. The phosphorus content of the inorganic phosphorus compound contained in the fatty acid chloride is preferably 0.15% by weight or less, and more preferably 0.10% by weight or less. Also, when the phosphorus content of the inorganic phosphorus compound contained in the fatty acid chloride is less than 0.03% by weight, the effect for stabilizing hue with the lapse of time may become poor. The phosphorus content of the inorganic phosphorus compound contained in the fatty acid chloride is preferably 0.04% by weight or more, and more preferably 0.05% by weight or more.

Also, the phosphorus content of the organic phosphorus compound contained in the fatty acid chloride obtained by performing Step 3 is from 0.04 to 0.10% by weight. The phosphorus content exceeding 0.10% by weight cause turbidity or purity degradation of the fatty acid chloride. The phosphorus content is preferably 0.09% by weight or less, and more preferably 0.08% by weight or less. Also, when the phosphorus content of the organic phosphorus compound contained in the fatty acid chloride obtained by performing Step 3 is less than 0.04% by weight, the effect for stabilizing hue with the lapse of time may become poor. The phosphorus content of the organic phosphorus compound contained in the fatty acid chloride is preferably 0.05% by weight or more, and more preferably 0.06% by weight or more.

The inorganic phosphorus compound contained in the fatty acid chloride denotes a phosphorus compound which is distributed in a saturated saline solution layer in an oil-water separation operation using ethyl ether and a saturated saline solution, and includes, for example, phosphorus trichloride, phosphorous acid by-produced or a reaction product thereof. The phosphorus content of the inorganic phosphorus compound contained in the fatty acid chloride denotes a phosphorus content contained in the phosphorus compound which is distributed in the saturated saline solution layer in the oil-water separation operation using ethyl ether and a saturated saline solution.

The phosphorous acid by-produced in the fatty acid chloride denotes a phosphorus compound exclusive of phosphorus trichloride among the phosphorus compounds which are distributed in the saturated saline solution layer in the oil-water separation operation using ethyl ether and a saturated saline solution. The phosphorus content of the phosphorous acid by-produced in the fatty acid chloride denotes a value obtained by subtracting a phosphorus content of phosphorus trichloride determined by gas chromatography from a phosphorus content contained in the phosphorus compound distributed in the saturated saline solution in the oil-water separation operation using ethyl ether and a saturated saline solution.

The organic phosphorus compound contained in the fatty acid chloride denotes a phosphorus compound which is distributed in an ethyl ether layer in the oil-water separation operation using ethyl ether and a saturated saline solution, and is mainly composed of a reaction product of an inorganic phosphorus compound, for example, phosphorus trichloride, with the fatty acid and the fatty acid chloride. The phosphorus content of the organic phosphorus compound contained in the fatty acid chloride denotes a phosphorus content contained in the phosphorus compound which is distributed in the ethyl ether layer in the oil-water separation operation using ethyl ether and a saturated saline solution.

EXAMPLE

(Measuring method of phosphorus content)

Preparation of calibration curve

As a standard sample, an aqueous solution corresponding to 2 μg/ml of phosphorus was prepared using monopotassium phosphate (special grade). The aqueous solution was aliquoted in a separatory funnel in appropriate amounts (several kinds of amounts between 0 to 70 μg) by a whole pipet, and the total volume was made up to 50 ml with water. 15 ml of a 10% aqueous nitric acid solution, 5 ml of a 5% ammonium molybdate solution and 10 ml of n-butyl acetate were added thereto, and the mixture was shaken for 3 minutes and then stood still. The lower layer was aliquoted in another separatory funnel, 10 ml of n-butyl acetate was added thereto, and the mixture was shaken for 3 minutes and then stood still. The n-butyl acetate layer in the separatory funnel was transferred to a 50 ml measuring flask. 2 ml of 3% stannous chloride solution was added thereto, and the volume was fixed with ethyl alcohol. Using a spectral photometer, the absorbance at 725 nm was measured (10 mm glass cell).

Pre-treatment method

To a separatory funnel (A) were added 50 ml of a saturated saline solution and 20 ml of ethyl ether, and from 0.1 to 0.5 g of a fatty acid chloride was weighed and added thereto. The separatory funnel (A) was shaken for 3 minutes and then stood still to separate layers. The saturated saline solution layer of the lower layer separated was aliquoted in another separatory funnel (B). To the separatory funnel (B) was added 20 ml of ethyl ether, and the separatory funnel (B) was shaken for 3 minutes and then stood still to separate layers. Also, to the separatory funnel (A) containing the remaining ethyl ether layer was added 25 ml of a saturated saline solution, and the separatory funnel (A) was shaken for 3 minutes and then stood still to separate layers. Then, the lower layers of the separatory funnel (A) and the separatory funnel (B) were aliquoted in the same conical beaker (C) to obtain the saturated saline solution layer. Also, the solutions remained in the separatory funnel (A) and the separatory funnel (B) were aliquoted in the same Kjeldahl flask (D) to obtain the ethyl ether layer.

Measuring method of phosphorus content of inorganic phosphorus compound

To the saturated saline solution layer in the conical beaker (C) were added 1 ml of a 10% aqueous nitric acid solution and 5 ml of a 2% potassium permanganate solution, and the mixture was heated at 200°C to oxidized phosphorus contained. After preparation of brown precipitates of manganese oxide oxidized, the heating was continued for about 10 minutes, and then a 10% sodium sulfite solution was dropwise added thereto to reduce. After cooling to room temperature, a few drops of bromphenol blue indicator were added, and neutralized with a 14% aqueous ammonia. The solution was transferred to a 200 ml measuring flask, and the volume was fixed with water. 50 ml of the solution was aliquoted in a separatory funnel by a whole pipet. 15 ml of a 10% aqueous nitric acid solution, 5 ml of a 5% ammonium molybdate solution and 10 ml of n-butyl acetate were added thereto, and the mixture was shaken for 3 minutes and then stood still. The lower layer was aliquoted in another separatory funnel, 10 ml of n-butyl acetate was added thereto, and the mixture was shaken for 3 minutes and then stood still. The n-butyl acetate layer in the separatory funnel was transferred to a 50 ml measuring flask. 2 ml of a 3% stannous chloride solution was added thereto, and the volume was fixed with ethyl alcohol. Using a spectral photometer, the absorbance at 725 nm was measured (10 mm glass cell) . The phosphorus content was obtained from the calibration curve previously prepared.

Also, a blank experiment was performed in parallel with the experiment.

Measuring method of phosphorus content of organic phosphorus compound

The ethyl ether in the Kjeldahl flask (D) was completely distilled off. 5 ml of sulfuric acid was added thereto, and carbonization was conducted by a Kjeldahl cracking unit. After cooling the inside of the flask to room temperature, about 5 ml of a hydrogen peroxide solution was gradually added thereto from a dropping funnel, and decomposition was conducted by a Kjeldahl cracking unit. Then, about 15 ml of a hydrogen peroxide solution was continuously added dropwise from a dropping funnel at a rate of about 1.5 ml per minute. The solution was concentrated to expel almost the hydrogen peroxide solution, and after generating white smoke of sulfuric acid, the solution turned colorless and transparent. After allowing to cool to room temperature, 50 ml of water and 1 ml of a 2% potassium permanganate solution were added thereto, decomposition of the hydrogen peroxide and oxidation were performed at the same time in the Kjeldahl cracking unit to prepare brown precipitates of manganese oxide, then the heating was continued for about 10 minutes, and then a 10% sodium sulfite solution was dropwise added thereto to reduce. After cooling to room temperature, a few drops of bromphenol blue indicator were added, and neutralized with a 14% aqueous ammonia. The solution was transferred to a 200 ml measuring flask, and the volume was fixed with water. 50 ml of the solution was aliquoted in a separatory funnel by a whole pipet. 15 ml of a 10% aqueous nitric acid solution, 5 ml of a 5% ammonium molybdate solution and 10 ml of n-butyl acetate were added thereto, and the mixture was shaken for 3 minutes and then stood still. The lower layer was aliquoted in another separatory funnel, 10 ml of n-butyl acetate was added thereto, and the mixture was shaken for 3 minutes and then stood still. The n-butyl acetate layer in the separatory funnel was transferred to a 50 ml measuring flask. 2 ml of a 3% stannous chloride solution was added thereto, and the volume was fixed with ethyl alcohol. Using a spectral photometer, the absorbance at 725 nm was measured (10 mm glass cell) . The phosphorus content was obtained from the calibration curve previously prepared.

Also, a blank experiment was performed in parallel with the experiment.

```
Phosphorus content (% by weight) of inorganic phosphorus

compound =

    (Phosphorus  content  (g)  obtained  from  calibration

curve/Sample collection quantity (g)) × Distillation rate × 100

        Phosphorus content (% by weight) of organic phosphorus

compound =

    (Phosphorus  content  (g)  obtained  from  calibration

curve/Sample collection quantity (g)) × Distillation rate × 100
```

Measuring method of phosphorus content of phosphorus trichloride

One g of acid chloride was weighed and about 3 ml of n-butanol was added thereto to convert a butyl ester (A). Also, as a correction criterion of implant volume, using a prescribed amount of butyl stearate the volume was fixed at 220 ml with n-butanol to prepare a solution. One ml of the solution was added to a 10 ml measuring flask using a whole pipet (B). Then, the solution of (A) was transferred to the 10 ml measuring flask of (B) while washing with about 2 ml of n-butanol and the volume was fixed at 10 ml with n-butanol. To the mixed solution was added the same volume of water, and the upper layer was measured by gas chromatography. From the peak area obtained, the amount of phosphorus trichloride in the acid chloride was determined by using a calibration curve of phosphorus trichloride previously prepared and then, the phosphorus content of phosphorus trichloride was calculated (phosphorus content of phosphorus trichloride = calculated value × (phosphorus /phosphorus trichloride)).

Measuring method of phosphorus content of phosphorous acid by-produced

A value obtained by subtracting the phosphorus content of phosphorus trichloride from the phosphorus content of inorganic phosphorus compound is defined as a phosphorus content of the phosphorous acid by-produced.

Example 1: Palm oil fatty acid chloride

To palm oil fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid by-produced (57.0 g) of the lower layer was removed to obtain a reaction solution (473.0 g). Subsequently, a topping treatment was performed using a thin film distiller having a heat transfer area of 0.03 m$^2$ at 70°C, 665 Pa and a flow rate of 300 g/hr to remove the unreacted phosphorus trichloride, thereby obtaining palm oil fatty acid chloride (451.7 g). The phosphorus content of the phosphorous acid by-produced after the topping treatment was 0.04% by weight. Then, phosphorus trichloride (0.8 g) was added thereto so as to set the phosphorus content thereof to 0.04% by weight, thereby obtaining palm oil fatty acid chloride (452.5 g). In the palm oil fatty acid chloride obtained, the phosphorus content of the organic phosphorus compound was 0.08% by weight, and the phosphorus content of the inorganic phosphorus compound was 0.07% by weight.

Example 2: Lauric acid chloride

To lauric acid (400.0 g) was added dropwise 1.5/3 equivalent of phosphorus trichloride (130.7 g) at from 50 to 60°C to perform a chlorination reaction. After standing still for 2 hours to separate layers, phosphorous acid by-produced (58.2 g) of the lower layer was removed to obtain a reaction solution (472.5 g). Subsequently, a topping treatment was performed using a thin film distiller having a heat transfer area of 0.03 m$^2$ at 85°C, 133.3 × 10 Pa and a flow rate of 300 g/hr to remove the unreacted phosphorus trichloride, thereby obtaining lauric acid chloride (451.2 g). The phosphorus content of the phosphorous acid by-produced after the topping treatment was 0.03% by weight. Then, phosphorus trichloride (1.2 g) was added thereto so as to set the phosphorus content thereof to 0.06% by weight, thereby obtaining lauric acid chloride (452.4 g). In the lauric acid chloride obtained, the phosphorus content of the organic phosphorus compound was 0.10% by weight, and the phosphorus content of the inorganic phosphorus compound was 0.06% by weight.

Example 3: Palm oil fatty acid chloride

To palm oil fatty acid (400.0 g) was added dropwise 1.8/3 equivalent of phosphorus trichloride (156.0 g) at from 50 to 60°C to perform a chlorination reaction. After standing still for 2 hours to separate layers, phosphorous acid by-produced (57.1 g) of the lower layer was removed to obtain a reaction solution (498.9 g). Subsequently, a topping treatment was performed using a thin film distiller having a heat transfer area of 0.03 m$^2$ at 60°C, 266.6 Pa and a flow rate of 300 g/hr to remove the unreacted phosphorus trichloride, thereby obtaining palm oil fatty acid chloride (476.4 g). The phosphorus content of the phosphorous acid by-produced after the topping treatment was 0.17% by weight. Then, phosphorus trichloride (0.6 g) was added thereto so as to set the phosphorus content thereof to 0.03% by weight, thereby obtaining palm oil fatty acid chloride (477.0 g). In the palm oil fatty acid chloride obtained, the phosphorus content of the organic phosphorus compound was 0.09% by weight, and the phosphorus content of the inorganic phosphorus compound was 0.20% by weight.

Example 4: Stearic acid chloride

To stearic acid (435.0 g) was added dropwise 2.0/3 equivalent of phosphorus trichloride (131.0 g) at from 60 to 65°C to perform a chlorination reaction. After standing still for 2 hours to separate layers, phosphorous acid by produced (45.0 g) of the lower layer was removed to obtain a reaction solution (521.0 g). Subsequently, a distillation treatment was performed using a thin film distiller having a heat transfer area of 0.03 m$^2$ at 220°C, 665 Pa and a flow rate of 200 g/hr to obtain stearic acid chloride (442.9 g). The phosphorus content of the phosphorous acid by-produced after the distillation treatment was N. D. (less than 0.01% by weight). Then, phosphorus trichloride (1.0 g) was added thereto so as to set the phosphorus content thereof to 0.05% by weight, thereby obtaining stearic acid chloride (443.9 g). In the stearic acid chloride obtained, the phosphorus content of the organic phosphorus compound was 0.08% by weight, and the phosphorus content of the inorganic phosphorus compound was 0.03% by weight.

Example 5: Palm oil fatty acid chloride

To palm oil fatty acid (400 g) was added dropwise 1/3 equivalent of phosphorus trichloride (86.7 g) at from 50 to 60°C to perform a chlorination reaction. After standing still for 2 hours to separate layers, phosphorous acid (56.6 g) of the lower layer was removed to obtain a reaction solution (455.4 g). Subsequently, a distillation treatment was performed using a thin film distiller having a heat transfer area of 0.03 m$^2$ at 140°C, 665 Pa and a flow rate of 200 g/hr to obtain palm oil fatty acid chloride (432.0 g). The phosphorus content of the phosphorous acid by-produced after the distillation treatment was N. D. (less than 0.01% by weight). Then, phosphorus trichloride (1.0 g) was added thereto so as to set the phosphorus content thereof to 0.05% by weight, thereby obtaining palm oil fatty acid chloride (443.0 g). In the palm oil fatty acid chloride obtained, the phosphorus content of the organic phosphorus compound was 0.04% by weight, and the phosphorus content of the inorganic phosphorus compound was 0.03% by weight.

Example 6: Palm oil fatty acid chloride

To palm oil fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid by-produced (57.0 g) of the lower layer was removed to obtain a reaction solution (473.0 g). Subsequently, a distillation treatment was performed using a thin film distiller having a heat transfer area of 0.03 m$^2$ at 140°C, 266 Pa and a flow rate of 200 g/hr to obtain palm oil fatty acid chloride (452.6 g). The phosphorus content of the phosphorous acid by-produced after the distillation treatment was 0.01% by weight. Then, phosphorus trichloride (1.0 g) was added thereto so as to set the phosphorus content thereof to 0.05% by weight, thereby obtaining palm oil fatty acid chloride (453.6 g). In the palm oil fatty acid chloride obtained, the phosphorus content of the organic phosphorus compound was 0.08% by weight, and the phosphorus content of the inorganic phosphorus compound was 0.03% by weight.

Comparative Example 1: Stearic acid chloride

To stearic acid (435.0 g) was added dropwise 2.0/3 equivalent of phosphorus trichloride (131.0 g) at from 60 to 65°C to perform a chlorination reaction. After standing still for 2 hours to separate layers, phosphorous acid by-produced (45.0 g) of the lower layer was removed to obtain a reaction solution (521.0 g) . The phosphorus content of the phosphorous acid by-produced after synthesis was 0.40% by weight. In the stearic acid chloride obtained, the phosphorus content of the organic phosphorus compound was 0.04% by weight, and the phosphorus content of the inorganic phosphorus compound was 0.90% by weight.

Comparative Example 2: Palm oil fatty acid chloride

To palm oil fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid by-produced (57.5 g) of the lower layer was removed to obtain a reaction solution (472.5 g). Subsequently, a distillation treatment was performed using a thin film distiller having a heat transfer area of $0.03 \text{ m}^2$ at 135°C, 133.3 Pa and a flow rate of 100 g/hr to obtain palm oil fatty acid chloride (401.6 g). The phosphorus content of the phosphorous acid by-produced after the distillation treatment was N. D. (less than 0.01% by weight). In the palm oil fatty acid chloride obtained, the phosphorus content of the organic phosphorus compound was 0.01% by weight, and the phosphorus content of the inorganic phosphorus compound was N. D. (less than 0.01% by weight).

Comparative Example 3: Lauric acid chloride

To lauric acid (400.0 g) was added dropwise 2.5/3 equivalent of phosphorus trichloride (218.0 g) at from 50 to 60°C to perform a chlorination reaction. After standing still for 2 hours to separate layers, phosphorous acid by-produced (58.2 g) of the lower layer was removed to obtain a reaction solution (559.8 g). Subsequently, a topping treatment was performed using a thin film distiller having a heat transfer area of $0.03 \text{ m}^2$ at 65°C, $133.3 \times 10$ Pa and a flow rate of 300 g/hr to remove the unreacted phosphorus trichloride, thereby obtaining lauric acid chloride (534.6 g). The phosphorus content of the phosphorous acid by-produced after the topping treatment was 0.40% by weight. Then, phosphorus trichloride (1.0 g) was added thereto so as to set the phosphorus content thereof to 0.04% by weight, thereby obtaining lauric chloride (535.6 g). In the lauric acid chloride obtained, the phosphorus content of the organic phosphorus compound was 0.12% by weight, and the phosphorus content of the inorganic phosphorus compound was 0.53% by weight.

Comparative Example 4: Palm nucleus fatty acid chloride

To palm nucleus fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid by-produced (57.0 g) of the lower layer was removed to obtain palm nucleus fatty acid chloride (473.0 g). Subsequently, a topping treatment was performed using a thin film distiller having a heat transfer area of $0.03 \text{ m}^2$ at 80°C, $200 \times 10^2$ Pa and a flow rate of 100 g/hr to remove the unreacted phosphorus trichloride, thereby obtaining palm nucleus fatty acid chloride (451.7 g). The phosphorus content of the phosphorous acid by-produced after the topping treatment was 0.09% by weight. Then, phosphorus trichloride (0.8 g) was added thereto so as to set the phosphorus content thereof to 0.04% by weight, thereby obtaining palm nucleus fatty acid chloride (452.5 g). In the palm nucleus fatty acid chloride obtained, the phosphorus content of the organic phosphorus compound was 0.22% by weight, and the phosphorus content of the inorganic phosphorus compound was 0.13% by weight.

Comparative Example 5: Lauric acid chloride

To lauric acid (400.0 g) was added dropwise 1.5/3 equivalent of phosphorus trichloride (130.0 g) at from 50 to 60°C to perform a chlorination reaction. After standing still for 2 hours to separate layers, phosphorous acid by-produced (57.0 g) of the lower layer was removed to obtain lauric acid chloride (473.0 g). Subsequently, a topping treatment was performed using a thin film distiller having a heat transfer area of $0.03 \text{ m}^2$ at 70°C, 133 Pa and a flow rate of 300 g/hr to remove the unreacted phosphorus trichloride, thereby obtaining lauric acid chloride (451.7 g). The phosphorus content of the phosphorous acid by-produced after the topping treatment was 0.10% by weight. Then, phosphorus trichloride (4.5 g) was added thereto so as to set the phosphorus content thereof to 0.22% by weight, thereby obtaining lauric acid chloride (456.2 g). In the lauric acid chloride obtained, the phosphorus content of the organic phosphorus compound was 0.09% by weight, and the phosphorus content of the inorganic phosphorus compound was 0.28% by weight.

Comparative Examples 6 to 12

To the distilled palm oil fatty acid chloride of Comparative Example 2 were added phosphonic acid (special grade, produced by Wako Pure Chemical Industries, Ltd.) and dodecyl phosphate (produced by Wako Pure Chemical Industries, Ltd.), and the phosphorus contents of the inorganic phosphorus compound and the organic phosphorus compound were measured.

(Evaluation of turbidity of fatty acid chloride)

Each of the fatty acid chloride solutions obtained was put into a 100 ml sample bottle made of glass, observed its appearance at 25°C, and evaluated according to the criteria shown below.

○○: Transparent
○: Slightly turbid
Δ: Turbid
✕: Precipitated

(Evaluation of hue of fatty acid chloride (temporal stability))

Each of the fatty acid chlorides obtained was put into a 100 ml sample bottle made of glass, the bottle was lidded and stored at 25°C or 40°C for one month, and change of hue (ΔAPHA) was evaluated.

$$\Delta\text{APHA} = (\text{APHA value after temporal stability test}) - (\text{APHA value before temporal stability test})$$

○○: ΔAPHA is from 0 to 29
○: ΔAPHA is from 30 to 59
Δ: ΔAPHA is from 60 to 89
✕: ΔAPHA is 90 or more

Table 1

|  | Example 1 | Example 2 | Example 3 | Example 4 | Example 5 | Example 6 |
|---|---|---|---|---|---|---|
| Charging Ratio of Phosphorus Trichloride | 1.5/3 | 1.5/3 | 1.8/3 | 2.0/3 | 1/3 | 1.513 |
| Phosphorus Content of Organic Phosphorus Compound after Step 1 (% by weight) | 0.02 | 0.02 | 0.04 | 0.05 | 0.02 | 0.02 |
| Phosphorus Content of Inorganic Phosphorus Compound after Step 1 (% by weight) | 0.9 | 1 | 1.5 | 1.8 | 0.62 | 0.9 |
| Phosphorus Content of Organic Phosphorus Compound after Step 2 (% by weight) | 0.06 | 0.07 | 0.07 | 0.05 | 0.02 | 0.06 |

(continued)

| | | Example 1 | Example 2 | Example 3 | Example 4 | Example 5 | Example 6 |
|---|---|---|---|---|---|---|---|
| Phosphorus Content of Inorganic Phosphorus Compound after Step 2 (% by weight) | | 0.05 | 0.03 | 0.19 | N.D. | N. D. | 0.01 |
| Phosphorus Content of Phosphorus Trichloride after Step 2 (% by weight) | | 0.01 | N. D. | 0.02 | N.D. | N.D. | N.D. |
| Phosphorus Content of Phosphorous Acid By-produced after Step 2 (% by weight) | | 0.04 | 0.03 | 0.17 | N.D. | N.D. | 0.01 |
| Phosphorus Content of Phosphorus Trichloride Added in Step 3 (% by weight) | | 0.04 | 0.06 | 0.03 | 0.05 | 0.05 | 0.05 |
| Phosphorus Content of Organic Phosphorus Compound after Step 3 (% by weight) | | 0.08 | 0.1 | 0.09 | 0.07 | 0.04 | 0.08 |
| Phosphorus Content of Inorganic Phosphorus Compound after Step 3 (% by weight) | | 0.07 | 0.06 | 0.2 | 0.03 | 0.03 | 0.03 |
| Turbidity | | ○○ | ○○ | ○ | ○○ | ○○ | ○○ |
| Hue (lapse of time) | APHA Value before Temporal Stability Test | 40 | 60 | 20 | 10 | 10 | 10 |
| | APHA Value after Temporal Stability Test at 25°C | 50 | 75 | 60 | 30 | 30 | 30 |
| | Evaluation | ○○ | ○○ | ○ | ○○ | ○○ | ○○ |
| | Temporal Stability Test at 40°C | 70 | 95 | 75 | 50 | 60 | 40 |
| | Evaluation | ○ | ○ | ○ | ○ | ○ | ○ |

Table 2

| | Comparative Example 1 | Comparative Example 2 | Comparative Example 3 | Comparative Example 4 | Comparative Example 5 |
|---|---|---|---|---|---|
| Charging Ratio of Phosphorus Trichloride | 1.5/3 | 1.5/3 | 2.5/3 | 1.5/3 | 1.5/3 |
| Phosphorus Content of Organic Phosphorus Compound after Step 1 (% by weight) | 0.04 | 0.02 | 0.08 | 0.02 | 0.02 |
| Phosphorus Content of Inorganic Phosphorus Compound after Step 1 (% by weight) | 0.9 | 0.9 | 3 | 1 | 0.9 |

(continued)

| | | Comparative Example 1 | Comparative Example 2 | Comparative Example 3 | Comparative Example 4 | Comparative Example 5 |
|---|---|---|---|---|---|---|
| Phosphorus Content of Organic Phosphorus Compound after Step 2 (% by weight) | | - | 0.01 | 0.11 | 0.2 | 0.06 |
| Phosphorus Content of Inorganic Phosphorus Compound after Step 2 (% by weight) | | - | N. D. | 0.5 | 0.11 | 0.1 |
| Phosphorus Content of Phosphorus Trichloride after Step 2 (% by weight) | | - | N.D. | 0.1 | 0.02 | N.D. |
| Phosphorus Content of Phosphorous Acid By-produced after Step 2 (% by weight) | | - | N.D. | 0.4 | 0.09 | 0.05 |
| Phosphorus Content of Phosphorus Trichloride Added in Step 3 (% by weight) | | 0 | 0 | 0.04 | 0.04 | 0.22 |
| Phosphorus Content of Organic Phosphorus Compound after Step 3 (% by weight) | | - | 0.01 | 0.12 | 0.22 | 0.09 |
| Phosphorus Content of Inorganic Phosphorus Compound after Step 3 (% by weight) | | - | N.D. | 0.53 | 0.13 | 0.28 |
| Turbidity | | × | ○ | × | Δ | × |
| Hue (lapse of time) | APHA Value before Temporal Stability Test | 20 | 5 | 30 | 80 | 80 |
| | APHA Value after Temporal Stability Test at 25°C | 30 | 130 | 120 | 160 | 115 |
| | Evaluation | ○○ | × | × | Δ | ○ |
| | Temporal Stability Test at 40°C | 35 | 300 | 250 | 220 | 140 |
| | Evaluation | ○○ | × | × | × | Δ |

Table 3

| | Comparative Example 6 | Comparative Example 7 | Comparative Example 8 | Comparative Example 9 | Comparative Example 10 | Comparative Example 11 | Comparative Example 12 |
|---|---|---|---|---|---|---|---|
| Phosphorus Content of Dodecyl Phosphate Added (% by weight) | 0.01 | 0.01 | 0.01 | 0.05 | 0.07 | 0.1 | 0.08 |
| Phosphorus Content of Phosphonic Acid Added (% by weight) | 0.05 | 0.1 | 0.27 | 0 | 0 | 0 | 0.07 |
| Phosphorus Content of Organic Phosphorus Compound (% by weight) | 0.01 | 0.01 | 0.01 | 0.05 | 0.07 | 0.1 | 0.08 |
| Phosphorus Content of Inorganic Phosphorus Compound (% by weight) | 0.1 | 0.05 | 0.27 | N. D. | N. D. | N. D. | 0.07 |
| Turbidity | ○○ | ○ | Δ | ○○ | ○○ | ○ | ○ |
| Hue (lapse of time) (25°C) | Evaluation | × | × | Δ | × | Δ | Δ | Δ |

In the examples shown in Table 1, the turbidity of the fatty acid chloride is small and the change of hue due to the lapse of time is inhibited.

In Comparative Example 1, Steps 2 and 3 are not performed, the phosphorus content of the inorganic phosphorus compound is large, and the precipitates are observed.

In Comparative Example 2, Step 3 is not performed, the phosphorus content of the inorganic phosphorus compound and the phosphorus content of the organic phosphorus compound are small, and the change of hue due to the lapse of time is large.

In Comparative Example 3, the charging ratio of phosphorus trichloride in Step 1 is large, the phosphorus content of the phosphorous acid by-produced in Step 2 is large, the phosphorus content of the organic phosphorus compound and phosphorus content of the inorganic phosphorus compound are large, the precipitates are observed, and the change of hue due to the lapse of time is large.

In Comparative Example 4, the pressure in Step 2 is high, the phosphorus content of the organic phosphorus compound is large, the turbidity is recognized, and the change of hue due to the lapse of time is large.

In Comparative Example 5, the addition amount of phosphorus trichloride in Step 3 is large, the phosphorus content of the inorganic phosphorus compound is large, the precipitates are observed, and the change of hue due to the lapse of time is observed.

In Comparative Examples 6 to 12, the phosphorus content of the inorganic phosphorus compound and the phosphorus content of the organic phosphorus compound are adjusted by adding the inorganic phosphorus compound or the organic phosphorus compound from outside to the furry acid chloride of Comparative Example 2 in which the phosphorus has been removed by distillation.

In Comparative Examples 6 to 8, the phosphorus content of the organic phosphorus compound is small, and the change of hue due to the lapse of time is large.

In Comparative Examples 9 to 11, the phosphorus content of the inorganic phosphorus compound is small, and the change of hue due to the lapse of time is large.

In Comparative Example 12, although both the phosphorus content of the inorganic phosphorus compound and the phosphorus content of the organic phosphorus compound are not different from those of the examples according to the invention, the change of hue due to the lapse of time is large.

## Claims

1. A production method of a fatty acid chloride comprising performing the following Step 1, Step 2 and Step 3 in this order:

   Step 1:
   a step of reacting a fatty acid having from 8 to 22 carbon atoms with from 1/3 to 2/3 molar equivalent of phosphorus trichloride relative to the fatty acid to prepare a fatty acid chloride, and separating, by standing, a layer containing phosphorous acid produced as a by-product to obtain a reaction product;
   Step 2:
   a step of purifying the reaction product obtained in the Step 1 so as to set a phosphorus content of the phosphorous acid by-produced to 0.20% by weight or less;
   Step 3:

   a step of adding from 0.03 to 0.07% by weight of phosphorus trichloride in terms of a phosphorus content to the purified product obtained in the Step 2,
   wherein in the Step 2, the purification is performed by treating the reaction product obtained in the Step 1 at a temperature from 80 to 230°C and under a pressure from $133.3 \times 10^{-1}$ to $133.3 \times 10^1$ Pa using a thin film distiller to distil away the phosphorus trichloride in the reaction product outside a system and to evaporate the fatty acid chloride and condense the fatty acid chloride by an internal condenser, thereby separating the phosphorous acid produced as a by-product which is a non-evaporative component, or
   wherein in the Step 2, the purification is performed by treating the reaction product obtained in the Step 1 at a temperature from 40 to 90°C and under a pressure from $133.3 \times 10^{-1}$ to $133.3 \times 10^1$ Pa using a thin film distiller to distil away the phosphorus trichloride in the reaction product outside a system without evaporating the fatty acid chloride, thereby removing the phosphorous acid produced as a by-product dissolved in the phosphorus trichloride together with the distillation away of the phosphorus trichloride from the fatty acid chloride,
   wherein the phosphorus content is measured in accordance with the conditions as described in the description.

**2.** The method as claimed in Claim 1, wherein a phosphorus content of an inorganic phosphorus compound contained in the fatty acid chloride obtained in the Step 3 is from 0.03 to 0.27% by weight and a phosphorus content of an organic phosphorus compound contained in the fatty acid chloride obtained in the Step 3 is from 0.04 to 0.10% by weight.

**3.** The method as claimed in Claim 1 or 2, wherein a phosphorus content of an inorganic phosphorus compound contained in the fatty acid chloride obtained in the Step 3 is from 0.05 to 0.10% by weight and a phosphorus content of an organic phosphorus compound contained in the fatty acid chloride obtained in the Step 3 is from 0.06 to 0.08% by weight.

**Patentansprüche**

**1.** Produktionsverfahren für ein Fettsäurechlorid, enthaltend die Durchführung des Schritts 1, des Schritts 2 und des Schritts 3 in dieser Reihenfolge:

Schritt 1:
einen Schritt der Reaktion einer Fettsäure mit 8 bis 22 Kohlenstoffatomen mit 1/3 bis 2/3 Moläräquivalent Phosphortrichlorid in Bezug auf die Fettsäure, zur Herstellung eines Fettsäurechlorids und Trennen einer Schicht, die Phosphorsäure enthält, die als Nebenprodukt erzeugt ist, durch Stehenlassen, unter Erhalt eines Reaktionsproduktes;
Schritt 2:
einen Schritt zur Reinigung des Reaktionsproduktes, erhalten im Schritt 1, um einen Phosphorgehalt der als Nebenprodukt erzeugten Phosphorsäure auf 0,20 Gew.-% oder niedriger einzustellen;
Schritt 3:

einen Schritt der Zugabe von 0,03 bis 0,07 Gew.-% Phosphortrichlorid, ausgedrückt als Phosphorgehalt, zu dem gereinigten Produkt, erhalten im Schritt 2,
worin im Schritt 2 die Reinigung durchgeführt wird durch Behandeln des im Schritt 1 erhaltenen Reaktionsproduktes bei einer Temperatur von 80 bis 230°C und unter einem Druck von $133,3 \times 10^{-1}$ bis $133,3 \times 10^1$ Pa durchgeführt wird, wobei ein Dünnfilmdestillator verwendet wird, zum Abdestillieren von Phosphortrichlorid im Reaktionsprodukt aus einem System heraus und zum Verdampfen des Fettsäurechlorides und Kondensieren des Fettsäurechlorides durch einen internen Kondensator, wodurch die Phosphorsäure, die als Nebenprodukt erzeugt ist, die eine nicht-verdampfende Komponente ist, getrennt wird, oder
worin im Schritt 2 die Reinigung durch Behandeln des Reaktionsproduktes, erhalten im Schritt 1, bei einer Temperatur von 40 bis 90°C und unter einem Druck von $133,3 \times 10^{-1}$ bis $133,3 \times 10^1$ Pa unter Verwendung eines Dünnfilmdestillators durchgeführt wird, zum Abdestillieren des Phosphortrichlorides im Reaktionsprodukt aus dem System heraus ohne Verdampfen des Fettsäurechlorides, unter Entfernung der Phosphorsäure, die als Nebenprodukt erzeugt ist, die im Phosphortrichlorid aufgelöst ist, zusammen mit der Destillation des Phosphortrichlorides von dem Fettsäurechlorid,
worin der Phosphorgehalt entsprechend den Bedingungen, die in dieser Beschreibung beschrieben sind, gemessen wird.

**2.** Verfahren gemäß Anspruch 1, worin ein Phosphorgehalt einer anorganischen Phosphorverbindung, die im Fettsäurechlorid enthalten ist, erhalten im Schritt 3, von 0,03 bis 0,27 Gew.-% ist und ein Phosphorgehalt einer organischen Phosporverbindung, die im Fettsäurechlorid enthalten ist, erhalten im Schritt 3, von 0,04 bis 0,10 Gew.-% ist.

**3.** Verfahren gemäß Anspruch 1 oder 2, worin ein Phosphorgehalt einer anorganischen Phosphorverbindung, die im Fettsäurechlorid enthalten ist, erhalten im Schritt 3, von 0,05 bis 0,10 Gew.-% ist und ein Phosphorgehalt einer organischen Phosporverbindung, die im Fettsäurechlorid enthalten ist, erhalten im Schritt 3, von 0,06 bis 0,08 Gew.-% ist.

**Revendications**

**1.** Procédé de production d'un chlorure d'acide gras comprenant la réalisation de l'étape 1, étape 2 et étape 3 suivantes dans cet ordre :

Étape 1 :

une étape consistant à faire réagir un acide gras ayant de 8 à 22 atomes de carbone avec de 1/3 à 2/3 d'équivalent molaire de trichlorure de phosphore par rapport à l'acide gras pour préparer un chlorure d'acide gras, et à séparer, en laissant au repos, une couche contenant de l'acide phosphoreux produit en tant que sous-produit pour obtenir un produit réactionnel ;

Étape 2 :

une étape consistant à purifier le produit réactionnel obtenu à l'étape 1 de sorte à fixer une teneur en phosphore du sous-produit d'acide phosphoreux à 0,20 % en poids ou moins ;

Étape 3 :

une étape consistant à ajouter de 0,03 à 0,07 % en poids de trichlorure de phosphore en terme de teneur en phosphore au produit purifié obtenu à l'étape 2,

dans lequel à l'étape 2, la purification est réalisée par traitement du produit réactionnel obtenu à l'étape 1 à une température de 80 à 230 °C et sous une pression de 133,3 x $10^{-1}$ à 133,3 x $10^1$ Pa en utilisant un distillateur à film mince pour séparer par distillation le trichlorure de phosphore dans le produit réactionnel à l'extérieur d'un système et pour évaporer le chlorure d'acide gras et condenser le chlorure d'acide gras par un condensateur interne, séparant ainsi l'acide phsophoreux produit en tant que sous-produit qui est un composant ne s'évaporant pas, ou

dans lequel à l'étape 2, la purification est réalisé par traitement du produit réactionnel obtenu à l'étape 1 à une température de 40 à 90 °C et sous une pression de 133,3 x $10^{-1}$ à 133,3 x $10^1$ Pa en utilisant un distillateur à film mince pour séparer par distillation le trichlorure de phosphore dans le produit réactionnel à l'extérieur d'un système sans évaporer le chlorure d'acide gras, éliminant ainsi l'acide phosphoreux produit en tant que sous-produit dissout dans le trichlorure de phosphore avec la séparation par distillation du trichlorure de phosphore du chlorure d'acide gras,

dans lequel la teneur en phosphore est mesurée selon les conditions telles que décrites dans la description.

2. Procédé selon la revendication 1, dans lequel une teneur en phosphore d'un composé phosphoré inorganique contenu dans le chlorure d'acide gras obtenu à l'étape 3 est de 0,03 à 0,27 % en poids et une teneur en phosphore d'un composé phosphoré organique contenu dans le chlorure d'acide gras obtenu à l'étape 3 est de 0,04 à 0,10 % en poids.

3. Procédé selon la revendication 1 ou 2, dans lequel une teneur en phosphore d'un composé phosphoré inorganique contenu dans le chlorure d'acide gras obtenu à l'étape 3 est de 0,05 à 0,10 % en poids et une teneur en phosphore d'un composé phosphoré organique contenu dans le chlorure d'acide gras obtenu à l'étape 3 est de 0,06 à 0,08 % en poids.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6041000 A
- JP 11255703 A
- WO 2005032509 A
- JP S63316753 A
- JP H01211547 A
- JP 2014058458 A